# EUROPEAN PATENT APPLICATION

(11) **EP 4 585 209 A1**
(43) Date of publication of application: **16.07.2025**
(21) Application number: 23895108.1
(22) Date of filing: 27.11.2023
(51) Int. Cl.: A61K 9/14, A61K 38/38, A61P 29/00, A61P 13/12

(54) **NANOPLATFORM FOR TARGETING INFLAMMATORY MACROPHAGES, AND COMPOSITION CONTAINING SAME FOR PREVENTING OR TREATING INFLAMMATORY DISEASES**

(30) Priority: 25.11.2022 KR 20220160450
(71) Applicant: Clichembio, Inc., Seoul 03122 (KR)
(72) Inventor: LEE, Yun Sang, Yongin-si Gyeonggi-do 16851 (KR); PARK, Ji Yong, Seoul 03724 (KR); YOO, Ran Ji, Seoul 01727 (KR); CHOI, Tae Hyeon, Seoul 02864 (KR); YANG, Seung Hee, Seoul 04593 (KR); KIM, Yon Su, Seoul 06001 (KR); KIM, Dong Ki, Seoul 06023 (KR); AN, Jung Nam, Anyang-si Gyeonggi-do 14102 (KR)
(74) Representative: AWA Sweden AB
(86) International application number: PCT/KR2023/019271
(87) International publication number: WO 2024/112181

(57) **Abstract**

The present invention relates to a nanoplatform for targeting an inflammatory macrophage and a composition for preventing or treating inflammatory diseases using the same. The nanoplatform for targeting the inflammatory macrophage according to the present invention can selectively target the inflammatory macrophage with a conjugated glucosyl group as a transmitter. Furthermore, it can suppress inflammatory responses in damaged cells or tissues, restore mitochondrial function, and inhibit cell death, making it an effective composition for preventing or treating inflammatory diseases, particularly kidney diseases.

## Description

### [Technical Field]

The present invention relates to a nanoplatform for targeting an inflammatory macrophage and a composition for preventing or treating inflammatory diseases using the same.

### [Background Art]

Inflammation refers to the expression of the body's defense mechanism against internal and external stimuli such as external infections and endogenous metabolic by-products through various pathways. Various intracellular inflammatory regulatory factors act as mediators in this process, contributing to the causes of various diseases such as allergies, atopy, arthritis, kidney disease, brain disorders, circulatory disorders, and even cancer.

Generally, the inflammatory response is a biological defense process aimed at repairing and regenerating damage caused by invasions that bring about structural changes to the body's cells or tissues. In this process, local blood vessels, various tissue cells in bodily fluids and immune cells are involved. While an inflammatory response induced by external invading pathogens serves as a defense system to protect the body under normal conditions, an abnormally excessive inflammatory response can lead to various diseases, which are collectively referred to as inflammatory diseases.

The onset of many inflammation-related diseases is associated with the activation of macrophages and the consequent excessive production of inflammation-related factors. Representative inflammatory factors include interleukin-1β (IL-1β), tumor necrosis factor-α (TNF-α), and nitrogen oxide (NO).

The kidney is an organ responsible for excreting waste products and maintaining homeostasis in the body. Kidney diseases occur due to a reduction in the filtration function of the glomeruli, the main structural units of the kidney, and can be broadly classified into acute kidney injury (AKI) and chronic kidney disease (CKD) based on the rate of kidney function impairment.

Acute kidney injury (AKI) refers to a rapid decline in kidney function occurring over a short period, such as a few hours or days. The prevalence of AKI is increasing annually regardless of gender or age and is observed in approximately 10% of hospitalized patients. Even if AKI patients recover, they may progress to chronic kidney disease (CKD) or end-stage renal disease (ESRD). If recovery does not occur, the risk of permanent kidney damage or mortality significantly increases. Chronic kidney disease (CKD) is a condition in which the glomerular filtration function gradually declines, ultimately leading to irreversible loss of kidney function. According to data tracked by the U.S. National Institutes of Health (NIH) until 2008, the risk of developing CKD increases in individuals with other illnesses. For instance, it has been reported that 1 in 3 diabetic patients and 1 in 5 hypertensive patients are affected by CKD.

Additionally, the number of patients with end-stage renal disease (ESRD) has shown a sharp increase over the past 20 years. In South Korea, the number of dialysis patients rose significantly from 64,679 in 2014 to 87,720 in 2019. ESRD patients impose a substantial burden on the healthcare system and society due to their high mortality rate and medical expenses.

Kidney fibrosis, a type of kidney disease, is considered the final common pathway. While early detection of the causes of kidney disease may allow recovery through appropriate treatment, if moderate or more severe chronic kidney disease (CKD) develops, it often progresses to end-stage renal disease (ESRD) or leads to death due to cardiovascular complications.

Proximal tubular epithelial cells, which are key components of kidney function, are characterized by a high density of mitochondria. In the pathophysiological background of kidney diseases such as acute kidney injury (AKI) and chronic kidney disease (CKD), mitochondrial alterations, damage, and dysfunction have been implicated. In particular, excessive activation of macrophages triggers inflammatory responses that can lead to mitochondrial damage. Prolonged mitochondrial damage induces oxidative stress, which in turn causes damage to proteins, lipids, and DNA in kidney cells, ultimately resulting in cellular apoptosis.

Given the growing recognition of the importance of mitochondria in kidney diseases, there is a pressing need to develop fundamental therapeutics capable of regulating the progression of kidney diseases by improving mitochondrial homeostasis and function.

### [Disclosure]

### [Technical Problem]

Object of the present invention is to provide an albumin-based nanoplatform that selectively targets inflammatory macrophages.

Another objective of the present invention is to provide a composition for preventing or treating inflammatory diseases, containing a nanoplatform for targeting inflammatory macrophages as an active ingredient.

Still another objective of the present invention is to provide a composition for preventing or treating kidney diseases, containing a nanoplatform for targeting inflammatory macrophages as an active ingredient.

### [Technical Solution]

In order to achieve the above object, the present disclosure provides a nanoplatform for targeting an inflammatory macrophage, the nanoplatform being obtained by a click chemistry reaction between albumin conjugated with an azide (N₃) or cyclooctyne functional group and a transmitter conjugated with an azide (N₃) or cyclooctyne functional group, wherein the transmitter comprises a glucosyl group, and when the albumin is conjugated with the azide functional group, the transmitter is conjugated with the cyclooctyne functional group, and when the albumin is conjugated with the cyclooctyne functional group, the transmitter is conjugated with the azide functional group.

In the present disclosure, the azide or cyclooctyne functional group introduced into the albumin may range from 1 to 14.

In the present disclosure, the nanoplatform may comprise 4 to 8 glucosyl groups.

In the present disclosure, the azide or cyclooctyne functional group may be conjugated to the 6th carbon of the glucosyl group.

In the present disclosure, the transmitter may further comprise a radioactive isotope, and the radioactive isotope may be one or more selected from the group consisting of H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re , ²²⁵Ac, ²¹²Pb, ^{117m}Sn and ¹⁷⁷Lu.

In the present disclosure, the radioactive isotope may be labeled by a chelating agent, and the chelating agent may be one or more selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihyroxy-7,10,18,21-tetraoxo-27-[N-acetylhydroxylamino)-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihyroxy-7,10,18,21-tetraoxo-27-[N-acetylhydroxylamino)-6,11,17,22-tetraazaheptaeicosane]thiourea) and HYNIC (hydrazinonicotinic acid).

In the present disclosure, the transmitter may further comprise a fluorescent material, and the fluorescent material may be one or more selected from the group consisting of FNR (Ferrodoxin NADP(+) reductase), cyanine-based fluorescent material, TAMRA (tetramethylrhodamine-5-maleimide), Flamma^{®} fluorescent material and ICG (indocyanine green).

In the present disclosure, the nanoplatform may selectively target a macrophage with overexpressed GLUT (Glucose Transporter).

The present disclosure also provides a pharmaceutical composition for preventing or treating an inflammatory disease, comprising the nanoplatform for targeting the inflammatory macrophage as an active ingredient.

The pharmaceutical composition for preventing or treating the inflammatory disease according to the present disclosure may selectively target a damaged tissue.

The pharmaceutical composition for preventing or treating the inflammatory disease according to the present disclosure may restore mitochondrial function within the damaged tissue and suppress cell death.

In the present disclosure, the inflammatory disease may be one or more selected from the group consisting of sepsis, septic shock, inflammatory bowel disease (IBD), peritonitis, inflammatory kidney disease, acute bronchitis, chronic bronchitis, osteoarthritis, enteropathic spondylitis, chronic obstructive pulmonary disease (COPD), rheumatoid arthritis, acute lung injury and bronchopulmonary dysplasia.

The present disclosure also provides a pharmaceutical composition for preventing or treating a kidney disease, comprising the nanoplatform for targeting the inflammatory macrophage as an active ingredient.

In the present disclosure, the kidney disease may be one or more selected from the group consisting of nephritis, pyelonephritis, nephrotic syndrome, kidney cancer, acute pyelonephritis, chronic pyelonephritis, renal tuberculosis, urinary tract infection, urolithiasis, ureteral calculus, acute renal failure, chronic renal failure, diabetic nephropathy, renal fibrosis, chronic glomerulonephritis, rapidly progressive glomerulonephritis, nephrotic syndrome, focal segmental glomerulosclerosis, membranous nephropathy and membranoproliferative glomerulonephritis.

### [Advantageous Effects]

The nanoplatform for targeting inflammatory macrophages according to the present disclosure is an albumin-based nanoplatform conjugated with a glucosyl group as a transmitter, which not only selectively targets inflammatory macrophages but also suppresses inflammatory responses in damaged cells or tissues, restores mitochondrial function and inhibits cell death. Therefore, it can be provided as an effective composition for preventing or treating inflammatory diseases, particularly inflammatory kidney diseases.

### [Description of Drawings]

FIG. 1 shows the results of verifying the targeting ability of glucosylated albumin and mannosylated albumin for M0, M1, and M2 types of macrophages according to one embodiment of the present disclosure.
FIG. 2 shows the results of verifying the targeting ability for GLUT-overexpressing cells based on albumin conjugation at the 1st, 2nd and 6th carbon positions of glucose according to one embodiment of the present disclosure.
FIG. 3 shows the results of verifying the cytotoxicity of glucosyl-conjugated albumin (Alb_Glu) in an acute kidney injury cell model according to one embodiment of the present disclosure.
FIG. 4 shows the results of verifying the effect of glucosylated albumin (Glu_Alb) on improving apoptosis/necrosis in an acute kidney injury cell model according to one embodiment of the present disclosure.
FIG. 5 shows fluorescence microscopy images verifying the effect of glucosylated albumin (Glu_Alb) on mitochondrial morphology restoration in an acute kidney injury cell model according to one embodiment of the present disclosure.
FIG. 6 shows fluorescence microscopy images verifying the effect of glucosylated albumin (Glu_Alb) on mitochondrial morphology restoration in an acute kidney injury cell model according to one embodiment of the present disclosure.
FIG. 7 shows the results of verifying the intracellular expression of glucosylated albumin (Glu_Alb) in an acute kidney injury cell model according to one embodiment of the present disclosure.
FIG. 8 shows a graph verifying the effect of glucosylated albumin (Glu_Alb) administration on improving kidney function in an ischemia-reperfusion injury (IRI) kidney damage animal model according to one embodiment of the present disclosure.
FIG. 9 shows the results of verifying the effect of glucosylated albumin (Glu_Alb) administration on improving kidney tissue and mitochondrial function in a kidney damage animal model according to one embodiment of the present disclosure.
FIG. 10 shows the results of verifying the levels of kidney damage and mitochondrial recovery in a kidney damage animal model administered with glucosylated albumin (Glu_Alb) according to one embodiment of the present disclosure.
FIG. 11 shows the results of verifying the targeting ability of glucosylated albumin labeled with radioisotopes to kidney tissue and kidney diseases in a bilateral ischemia-reperfusion injury (IRI) kidney damage animal model according to one embodiment of the present disclosure.
FIG. 12 shows the results of verifying the selective kidney disease-targeting ability of glucosylated albumin in a unilateral ischemia-reperfusion injury (IRI) kidney damage animal model according to one embodiment of the present disclosure.

### [Best Mode for Invention]

Hereinafter, the specific embodiments of the present disclosure will be described in more detail. Unless otherwise defined, all technical and scientific terms used herein have the same meanings as commonly understood by those skilled in the art to which the present disclosure pertains. In general, the nomenclature used herein is well-known and commonly used in the art.

The present disclosure relates to an albumin-based nanoplatform that selectively targets inflammatory macrophages and a composition for preventing or treating kidney diseases using the same.

Macrophages are broadly classified into inflammatory M1 type and anti-inflammatory M2 type. Depending on environmental stimuli, macrophages can undergo repolarization to change their type, and such type changes may directly contribute to pathogenesis. In particular, during the progression of inflammation, M1 macrophages play a key role in initiating the inflammatory response, which subsequently induces other immune cells and additional inflammatory reactions. Therefore, targeting M1 macrophages may be utilized for treating various autoimmune or other inflammatory diseases.

The present disclosure has discovered that a glucosylated albumin nanoplatform, which is albumin conjugated with glucose, not only selectively targets inflammatory macrophages but also improves macrophage and kidney function, thereby enabling the prevention or treatment of inflammatory diseases.

One aspect of the present disclosure provides a nanoplatform for targeting inflammatory macrophages, the nanoplatform being obtained by a click chemistry reaction between albumin conjugated with an azide (N₃) or cyclooctyne functional group and a transmitter conjugated with an azide (N₃) or cyclooctyne functional group, wherein the transmitter comprises a glucosyl group, and when the albumin is conjugated with the azide functional group, the transmitter is conjugated with the cyclooctyne functional group, and when the albumin is conjugated with the cyclooctyne functional group, the transmitter is conjugated with the azide functional group.

In the present disclosure, albumin refers to a protein that constitutes the basic materials of cells, is abundantly present in the blood, and is produced in the liver. The albumin has the lowest molecular weight among simple proteins that exist in nature. Serum albumin in the blood functions to maintain and restore plasma volume, preventing shock caused by excessive bleeding, and is used in surgery and burn treatments. It is also known to possess an oxygen transport capability similar to that of hemoglobin.

The albumin may include any albumin capable of being formulated, but may preferably be derived from human plasma or may be, but is not limited to, recombinant human serum albumin produced by genetic engineering. Genetic information regarding albumin in the present disclosure may be obtained from known databases, such as NCBI GenBank.

In the present disclosure, the number of amino groups (-NH₂) exposed on the surface of the albumin may range from 15 to 30.

In a specific embodiment of the present disclosure, human serum albumin (HSA), a representative biocompatible albumin, was used to develop a nanoplatform capable of targeting inflammatory macrophages in vivo and exhibiting therapeutic effects on diseases induced by inflammatory macrophages. Cyclooctyne or azide (N₃), one of the functional groups for click chemistry, was introduced onto the surface of HSA under reaction conditions that minimized HSA denaturation.

In the present disclosure, azide (N₃) is a reactive group composed of three nitrogen atoms and is known for its high reactivity. Specifically, it acts as an electron donor in a 1,3-dipolar cycloaddition reaction, a type of Cu-Free click chemistry, and plays a role in forming a triaza-5-membered ring.

The cyclooctyne functional group is an 8-membered aliphatic ring comprising a triple bond that undergoes ring strain. It is particularly known to act as an electron acceptor in a 1,3-dipolar cycloaddition reaction, a type of Cu-Free click chemistry, and plays a role in forming a triaza-5-membered ring. Due to the structural characteristics of cyclooctyne, specifically its triple bond structure under ring strain, click chemistry is possible without a Cu(I) catalyst.

The cyclooctyne functional group may be, but is not necessarily limited to, one or more selected from the group consisting of 4-cyclooctyn-1-yl 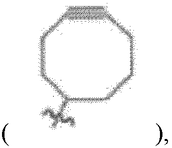 3-cyclooctyn-1-yl 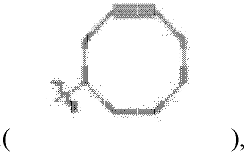 2-cyclooctyn-1-yl 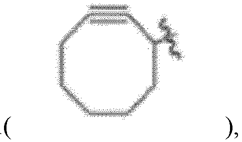 Monofluorinated cyclooctyne(MOFO) 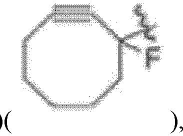 Difluororinated cyclooctyne(DIFO) 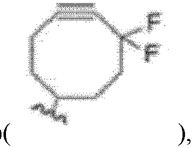 Dimethoxyazacyclooctyne(DIMAC) 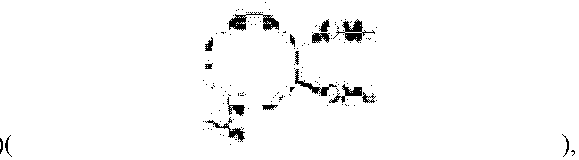 Dibenzocyclooctyne(DIBO) 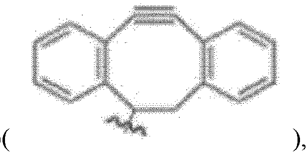 Azadibenzocyclooctyne(ADIBO) and biarylazacyclooctynone(BARAC)

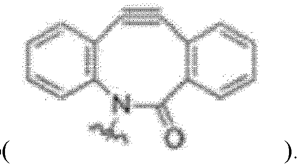

Albumin conjugated with the azide (N3) or cyclooctyne functional group may be obtained by the following steps: (a) dissolving albumin in a phosphate-buffered saline (PBS), (b) dissolving azide-NHS or cyclooctyne-NHS in DMSO, and (c) mixing the obtained solutions and then reacting them at 20 to 37°C for 30 minutes to 1 hour.

In step (a), the phosphate-buffered saline (PBS) may have a pH of 6.8 to 7.6, and preferably a pH of 7.0 to 7.4.

In step (b), the amount of DMSO used to prepare the azide-NHS solution or cyclooctyne-NHS solution may be 2% (v/v) or less of the total reaction solution.

In step (c), the molar mixing ratio of albumin to azide-NHS or cyclooctyne-NHS may range from 1:1 to 1:25.

In step (c), when mixing the albumin solution with the azide-NHS solution, the functional group conjugated to albumin may be the azide functional group, and when mixing the albumin solution with the cyclooctyne-NHS solution, the functional group conjugated to albumin may be the cyclooctyne functional group.

In one embodiment of the present disclosure, a human serum albumin (HSA) solution was mixed with an ADIBO-NHS solution and reacted at 37°C for 30 minutes to prepare HSA-ADIBO.

The number of click reaction functional groups (azide or cyclooctyne functional groups) introduced onto the surface of albumin is preferably 1 to 14, and more preferably 10 to 12. In this case, when infused into the human body, the functional groups can remain in the bloodstream for a long time and increase the likelihood of uptake at the target site. Conversely, if the number of click reaction functional groups is excessive, they may be immediately taken up by the liver upon administration, limiting uptake at other target disease sites. The number of click reaction functional groups introduced onto the albumin surface may be adjusted according to the reaction ratio of albumin to azide-NHS or cyclooctyne-NHS.

A nanoplatform for targeting inflammatory macrophages, which is an aspect of the present disclosure, is obtained by mixing a solution containing the aforementioned albumin conjugated with the azide (N₃) or cyclooctyne functional group and a solution containing a transmitter conjugated with the azide (N₃) or cyclooctyne functional group and performing a click chemistry reaction, wherein the click chemistry reaction may be a copper catalyst-free (Cu-free) click chemistry reaction.

In one aspect of the present disclosure, the azide functional group used as the click chemistry functional group acts as an electron donor, while the cyclooctyne functional group acts as an electron acceptor. Therefore, when the functional group conjugated to albumin is the azide functional group, it is preferable that the functional group conjugated to the transmitter is the cyclooctyne functional group. Conversely, when the functional group conjugated to albumin is the cyclooctyne functional group, it is preferable that the functional group conjugated to the transmitter is the azide functional group.

The nanoplatform for targeting inflammatory macrophages according to the present disclosure, having multiple click reaction functional groups, allows for the binding of various transmitters to multiple reactive sites.

In the present disclosure, the transmitter refers to a substance conjugated with albumin and delivered into the body, and it comprises a glucosyl group. The structure of the glucosyl group is shown in Formula 1 below:

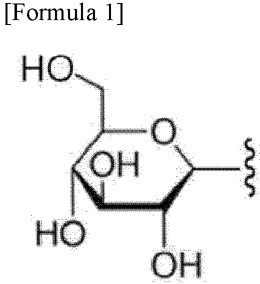

Macrophages, with their diversity and flexibility, can undergo repolarization in response to environmental stimuli, broadly transitioning into inflammatory M1 type, which defend against bacterial invasion, or M2 type, which are associated with tissue repair and anti-inflammatory responses. These macrophage type changes may directly contribute to the pathogenesis of diseases.

Among these, inflammatory M1 type macrophages exhibit overexpression of GLUT (Glucose Transporter) on their surface and rely exclusively on glycolysis for energy metabolism.

Accordingly, the albumin-based nanoplatform targeting for macrophages of the present disclosure, conjugated with a glucosyl group as a transmitter, possesses targeting ability toward inflammatory macrophages. Through this mechanism, it is selectively taken up by inflammatory macrophages in damaged tissues, suppressing inflammatory responses and ROS production induced by inflammatory macrophages. This restores the function of damaged mitochondria and inhibits cell death in damaged tissues. Thus, the nanoplatform may be utilized for preventing or treating progression to severe diseases

In particular, the present disclosure has confirmed that the type of macrophage targeted varies depending on the type of sugar attached to albumin. Specifically, albumin conjugated with a glucosyl group can selectively target M1 type macrophages. In one embodiment of the present disclosure, comparison experimental results between glucosyl-conjugated albumin and mannosyl-conjugated albumin demonstrated that glucosylated albumin exhibited high uptake efficiency in M1 type macrophages, whereas mannosylated albumin showed high uptake efficiency in M2 type macrophages.

In the present disclosure, the albumin-based nanoplatform for targeting inflammatory macrophages may comprise 4 to 8 glucosyl groups, and more preferably 5 to 7 glucosyl groups. In this range, the nanoplatform injected into the body can remain in the bloodstream for a long time and increase its targeting potential at the target site. However, if the number of glucosyl groups exceeds 8, the nanoplatform may be immediately taken up by the liver upon injection, significantly reducing the initial amount circulating in the blood, thereby limiting its targeting ability to the disease site.

In the present disclosure, the transmitter, glucosyl group, may be conjugated to the nanoplatform for targeting inflammatory macrophages via the click chemistry reaction between the azide or cyclooctyne functional group conjugated to albumin and the azide or cyclooctyne functional group conjugated to the glucosyl group.

At this time, GLUT, which is overexpressed on the surface of M1 type cells, recognizes the OH functional groups at the 1st, 3rd and 5th positions of glucose to obtain glucose as an energy source. In the present disclosure, to more effectively target M1 type macrophages, albumin may be conjugated using the 1st, 2nd and 6th carbon positions of glucose, and the 6th position is particularly preferred.

For example, glucosyl groups with the azide conjugated at the 1st, 2nd or 6th carbon position correspond to the chemical structures shown in Formula 2, Formula 3 and Formula 4, respectively:

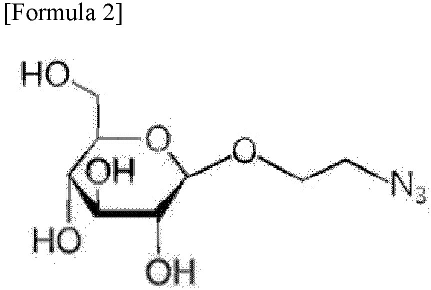

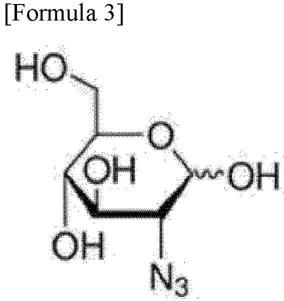

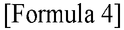

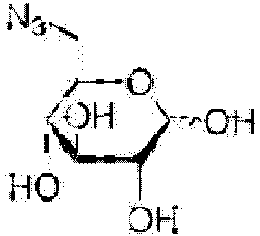

According to one embodiment of the present disclosure, after conjugating albumin to the 1st, 2nd or 6th carbon positions of glucose, the GLUT-overexpressing cell targeting ability was verified. The results showed that the nanoplatform with albumin conjugated to the 6th carbon position of glucose exhibited 3 times and 2 times higher GLUT targeting ability compared to the nanoplatforms with albumin conjugated to the 1st and 2nd carbon positions, respectively.

The albumin-based nanoplatform of the present disclosure may further comprise one or more substances in addition to the glucosyl group as a transmitter. In this case, multiple substances may simultaneously undergo the click chemistry reaction with albumin conjugated with the azide or cyclooctyne functional group, or each substance may sequentially undergo the click chemistry reaction.

In the present disclosure, the transmitter may further comprise a radioactive isotope in addition to the glucosyl group, wherein the radioactive isotope may be labeled by a chelating agent.

A radioactive isotope refers to an element with the same atomic number but a different atomic mass, and those that exhibit radioactivity are called radioactive isotopes. These radioactive isotopes may be used as critical markers for diagnosing diseases or verifying pharmacokinetics by utilizing their property of decaying radioactivity through the emission of gamma rays or other subatomic particles. The radioactive isotopes that may be used as markers in the present disclosure are not limited as long as they are known in the art, and may be, but are not limited to, one or more selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn and ¹⁷⁷Lu, and preferably ¹¹C, ¹⁸F, ⁶⁴Cu, ⁶⁷Ga, ⁶⁸Ga, ⁸⁹Zr, ^{99m}Tc, ¹¹¹In and ¹²³I.

A chelating agent serves to connect the radioactive isotope to albumin, and for example, may be, but is not limited to, one or more selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihydroxy-7,10,18,21-tetraoxo-27-[N-acetylhydroxylamino)-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihydroxy-7,10,18,21-tetraoxo-27-[N-acetylhydroxylamino)-6,11,17,22-tetraazaheptaeicosane]thiourea) and HYNIC (hydrazinonicotinic acid).

Fluorescent materials refer to materials that emit specific visible wavelengths in response to specific wavelengths of light. In the present disclosure, fluorescent materials that may bind to the albumin-based nanoplatform for targeting macrophages and be used to identify the location of the nanoplatform are all included.

Specifically, the fluorescent materials that may be used as markers in the present disclosure are not limited as long as they are known in the art, and the fluorescent material such as rhodamine series such as rhodamine, TAMRA (tetramethylrhodamine), etc.; fluorescein series such as fluorescein, FITC (fluorescein isothiocyanate), FAM (fluoresceinamidite), etc.; bodipy series (borondipyrromethene); alexa fluor series; and cyanine series such as Cy3, Cy5, Cy7, indocyanine green, etc., may be used, but are not limited thereto.

In the present disclosure, the transmitter may further comprise a fluorescent material.

Fluorescent materials refer to materials that emit specific visible wavelengths in response to specific wavelengths of light. In the present disclosure, fluorescent materials that may bind to the albumin-based nanoplatform for targeting macrophages and be used to identify the location of the nanoplatform are all included.

Specifically, the fluorescent materials that may be used as markers in the present disclosure are not limited as long as they are known in the art, and the fluorescent material such as rhodamine series such as rhodamine, TAMRA (tetramethylrhodamine), etc.; fluorescein series such as fluorescein, FITC (fluorescein isothiocyanate), FAM (fluoresceinamidite), etc.; bodipy series (borondipyrromethene); alexa fluor series; and cyanine series such as Cy3, Cy5, Cy7, indocyanine green, etc., may be used, but are not limited thereto.

Another aspect of the present disclosure relates to a pharmaceutical composition for preventing or treating inflammatory diseases, comprising the nanoplatform for targeting inflammatory macrophages as an active ingredient.

The present disclosure has discovered that an albumin-based nanoplatform conjugated with a glucosyl group can not only target inflammatory macrophages but also suppress inflammatory responses within tissues and restore mitochondrial function, thereby preventing or treating inflammatory diseases.

Specifically, the nanoplatform for targeting inflammatory macrophages according to the present disclosure can selectively target damaged tissues and be taken up into them. Once delivered to the damaged tissues, the nanoplatform is selectively taken up by inflammatory macrophages, thereby suppressing inflammatory responses within the tissues, restoring mitochondrial function and inhibiting cell death in the damaged tissues.

According to one embodiment of the present disclosure, the glucosylated albumin-based nanoplatform not only exhibits no cytotoxicity even at high concentrations but also demonstrates a significant improvement in apoptosis/necrosis compared to cells treated with albumin alone. Additionally, it shows superior efficacy in restoring mitochondrial morphology.

Therefore, the nanoplatform of the present disclosure may be utilized for preventing or treating inflammatory diseases. The inflammatory diseases may be one or more selected from the group consisting of sepsis, septic shock, inflammatory bowel disease (IBD), peritonitis, inflammatory kidney disease, acute bronchitis, chronic bronchitis, osteoarthritis, enteropathic spondylitis, chronic obstructive pulmonary disease (COPD), rheumatoid arthritis, acute lung injury and bronchopulmonary dysplasia.

The nanoplatform of the present disclosure may also be utilized for improving kidney diseases.

The glucosylated albumin-based nanoplatform of the present disclosure exhibits excellent targeting ability toward damaged kidney tissues and can significantly improve kidney function.

According to one embodiment of the present disclosure, a mouse model treated with the glucosylated albumin-based nanoplatform showed decreased expression of NGAL, cytochrome C and p21, along with increased expression of Sod-1 and E-cadherin, thereby demonstrating an improvement in kidney function.

In particular, the nanoplatform of the present disclosure remains in the body for a long time without being completely cleared even after 24 hours. This prolonged retention is expected to have a significant impact on disease treatment, such as pre-surgical administration.

The kidney disease in the present disclosure may by one or more selected from the group consisting of nephritis, pyelonephritis, nephrotic syndrome, kidney cancer, acute pyelonephritis, chronic pyelonephritis, renal tuberculosis, urinary tract infection, urolithiasis, ureteral calculus, acute renal failure, chronic renal failure, diabetic nephropathy, renal fibrosis, chronic glomerulonephritis, rapidly progressive glomerulonephritis, nephrotic syndrome, focal segmental glomerulosclerosis, membranous nephropathy and membranoproliferative glomerulonephritis, but is not necessarily limited thereto.

The pharmaceutical composition of the present disclosure may further comprise appropriate pharmaceutically acceptable carriers, excipients or diluents according to conventional methods. The pharmaceutically acceptable carriers are those commonly used in formulations and may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate and mineral oil.

The pharmaceutical composition of the present disclosure may further comprise lubricants, wetting agents, sweeteners, flavoring agents, emulsifiers, suspending agents, preservatives and the like. Suitable pharmaceutically acceptable carriers and formulation methods may be selected and formulated as appropriate for each component using methods disclosed in Remington's Pharmaceutical Sciences (19th ed., 1995).

The pharmaceutical composition of the present disclosure may be administered orally or parenterally. Parenteral administration includes intravenous, subcutaneous, intramuscular, intraperitoneal, transdermal administration and the like.

Oral formulations may include, for example, tablets, pills, hard or soft capsules, liquid solutions, suspensions, emulsions, syrups, granules and the like. These formulations may further comprise diluents (e.g., lactose, dextrose, sucrose, mannitol, sorbitol, cellulose and/or glycine) and lubricants (e.g., silica, talc, stearic acid and its magnesium or calcium salts and/or polyethylene glycol). Additionally, tablets may include binders such as magnesium aluminum silicate, starch paste, gelatin, tragacanth, methyl cellulose, sodium carboxymethyl cellulose and/or polyvinylpyrrolidone. They may also contain disintegrants such as starch, agar, alginic acid or its sodium salts, effervescent mixtures, and/or adsorbents, colorants, flavoring agents, and sweeteners. These formulations may be prepared using conventional mixing, granulating or coating methods.

Representative parenteral formulations include injectable preparations. Solvents for injectable preparations may include water, Ringer's solution, isotonic saline or suspensions.

Sterile fixed oils of the injectable preparations may also be used as solvents or suspension media, with any non-irritant fixed oil, including mono- and diglycerides, being suitable for this purpose. Fatty acids such as oleic acid may also be used.

The composition of the present disclosure is administered in a pharmaceutically effective amount. In the present disclosure, a "pharmaceutically effective amount" refers to an amount sufficient to treat a disease with a reasonable benefit/risk ratio applicable to medical treatment. The effective dosage level may depend on factors such as the type and severity of the disease, the activity of the drug, sensitivity to the drug, timing of administration, route of administration, excretion rate, treatment duration, concurrent medications and other factors well known in the medical field. The composition of the present disclosure may be administered as a single therapy or in combination with other therapies, either sequentially or simultaneously with conventional therapies, and may be administered in single or multiple doses. Considering all these factors, it is important to administer the minimum amount necessary to achieve maximum efficacy without side effects, which may be readily determined by a person skilled in the art.

Specifically, the effective dose of the composition of the present disclosure may vary depending on the patient's age, gender and weight. Generally, 0.001 to 150 mg per kg of body weight, preferably 0.01 to 100 mg, may be administered daily or every other day, or divided into 1 to 3 doses per day. However, the dosage may increase or decrease depending on the administration route, gender, body weight, age and other factors, and the specified dosage does not limit the scope of the present disclosure in any way.

The present disclosure will now be described in more detail through the following examples. These examples are provided to illustrate certain experimental methods and configurations and do not limit the scope of the present disclosure.

### Preparation Example 1: Preparation of Albumin-Based Nanoplatform for Targeting Macrophages

### 1-1. Preparation of Glucosyl-Conjugated Albumin

Albumin was dissolved in phosphate-buffered saline (PBS, pH 7.4) at a concentration of 40 mg/mL, and the albumin solution was aliquoted into vials at 20 mg/0.5 mL per vial. ADIBO-NHS was dissolved in DMSO (10 mg/50 µL) and added to the albumin solution to prepare a final volume of 500 µL.

Albumin and ADIBO-NHS were reacted at a ratio of 1:14 at room temperature for 6 hours and then purified using an Amicon Ultra-0.5 centrifugal filter tube. For glucose-albumin synthesis, 6-azido-glucose was dissolved in distilled water at a concentration of 3 mg/mL and added to the prepared ADIBO-albumin (AD-Alb) solution (4 mg/0.5 mL). The mixture was incubated at room temperature for 1 hour and then purified using a centrifugal filter tube.

Next, the absorbance at 280 nm and 309 nm was measured using UV-Vis spectroscopy to analyze the trend of Glucose-AD-Alb (Glc-AD-Alb). To measure the absorbance of albumin, each albumin sample was diluted to 2 mg/mL. The number of ADIBO and glucose molecules conjugated to albumin was determined using MALDI-TOF MS (degree of functionalization = DOF).

Subsequently, the number of ADIBO molecules on the albumin surface and the number of glucose molecules introduced through click reactions were quantified using UV and MALDI-TOF analyses. For the preparation of AD11-Alb-FL and Glc6-AD11-Alb-FL, N₃-FNR648 (10 nmol/1 µL, dissolved in DMSO) was added to AD-Alb and Glc-AD-Alb (30 nmol/500 µL), incubated at 4°C for 30 minutes, and purified using centrifugal filtration.

Quantification of the number of ADIBO functional groups introduced into HSA-ADIBO via UV-Vis spectrophotometry revealed that the number is 12.8 to 13.1, while MALDI-TOF analysis showed that the number is 11.1 to 11.5. Additionally, the number of conjugated glucose molecules was confirmed to be 5.4 to 7.8.

**[Table 1]**

| ADIBO DOF | | Glc DOF (MALDI-TOF) | Albumin Concentration (mg/gl) |
|---|---|---|---|
| UV | MLADI-TOF | | |
| 13.1 | 11.1 | 6.2 | 29.8 |
| 13.8 | 11.2 | 5.4 | 30.6 |
| 12.8 | 11.5 | 7.8 | 37.3 |

### 1-2. Preparation of Mannosyl-Conjugated Albumin

HSA-ADIBO was dissolved in PBS, and Man-N₃ (1-O-(2-(2-(2-azidoethoxy)ethoxy)ethoxy)-alpha-D-mannopyranoside) was mixed with HSA-ADIBO at a molar ratio of 1:8 (HSA-ADIBO:Man-N₃). The mixture was reacted at 37°C for 1 hour to produce mannosyl-conjugated albumin. The conjugation of the mannosyl group was subsequently confirmed using the method described in Preparation Example 1-1.

### 1-3. Conjugation of Fluorescent Materials or Radioactive Isotopes

A vial containing ⁶⁴Cu was dried under N₂ gas in a fume hood for 30 minutes. Subsequently, 200 µL of 1M sodium acetate buffer (pH 5) was added to the vial to adjust the pH to 5, followed by the addition of NOTA-N₃ (1 mg/mL in distilled water, 10 µL). The mixture was heated at 70°C for 5 minutes.

To measure the radiolabeling efficiency, thin-layer chromatography (TLC) was performed using ITLC-SG paper (0.1 M citrate as the mobile phase, Radio-TLC). After confirming the radiolabeling efficiency, a radiolabeled NOTA-N₃ solution ([⁶⁴Cu]Cu-NOTA-N3, 10 mCi/200 µL) was added to Glc6-AD11-Alb or AD11-Alb at a volume of 10 µL (9 nmol, 0.5 mCi).

Alb in PBS (500 µg/0.5 mL, 15 nmol/0.5 mL) was reacted at room temperature (RT) for 30 minutes, and then finally the Rf values of unbound ⁶⁴Cu and [⁶⁴Cu] were confirmed. To verify the generation of radiolabeled Glc6-AD11-Alb or AD11-Alb (Glc6-AD11-Alb-⁶⁴Cu or AD11-Alb-⁶⁴Cu), radio-TLC was performed. The Rf values for ⁶⁴Cu-NOTA-N₃, Glc6-AD11-Alb-⁶⁴Cu and AD11-Alb-⁶⁴Cu were determined to be 1.0, 0.5-0.6 and 0.0-0.1, respectively.

### Experimental Example 1: Verification of Macrophage Targeting Ability of Albumin-Based Nanoplatform

An experiment was conducted to verify the macrophage targeting ability of HSA-ADIBO conjugated with glucosyl and mannosyl groups, as obtained in Preparation Example 1.

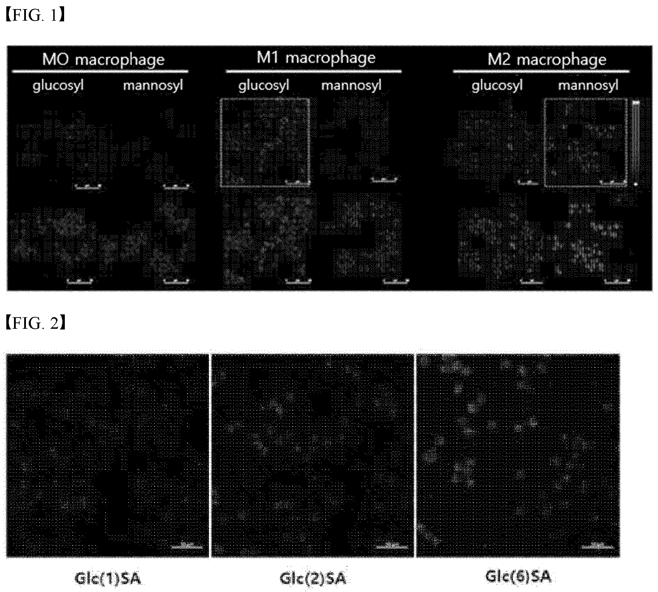

As shown in FIG. 1, glucosylated albumin exhibited a high uptake rate in M1 macrophages, while mannosylated albumin demonstrated a high uptake effect in M2 macrophages. Both glucosylated and mannosylated albumins showed scarce uptake in M0 macrophages.

These results indicate that the type of sugar conjugated to the albumin surface influences macrophage targeting ability. Furthermore, the glucosylated albumin-based nanoplatform of the present disclosure was confirmed to selectively target inflammatory M1 macrophages.

### Experimental Example 2: Verification of Targeting Ability Based on Glucose Carbon Position

An experiment was conducted to verify the macrophage targeting ability based on the carbon position of glucose and to determine the optimal carbon position for albumin conjugation.

For this purpose, glucosylated albumins with albumin conjugated to the 1st, 2nd or 6th carbon positions of glucose were treated in MDA-MB231 cancer cells, which exhibit low expression of SPARC (the albumin uptake mechanism) but overexpress GLUT, to assess targeting ability.

As shown in FIG. 2, significant differences in the targeting ability of GLUT-overexpressing cells were observed depending on the carbon position of glucose. Notably, the nanoplatform with albumin conjugated at the 6th carbon position of glucose demonstrated the highest GLUT targeting ability. Specifically, the targeting ability of the nanoplatform with albumin conjugated at the 6th position was three times higher than that of the nanoplatform with albumin conjugated at the 1st position and two times higher than that of the nanoplatform with albumin conjugated at the 2nd position.

### Experimental Example 3: Verification of the In Vitro Kidney Function Improvement Effect of the Albumin-Based Nanoplatform

The kidney function improvement effect of the glucosyl group conjugated albumin nanoplatform (glucose-albumin) was evaluated in an acute kidney injury (AKI) cell model using human primary cultured renal proximal tubular epithelial cells.

An AKI cell model was prepared by treating human primary cultured renal proximal tubular epithelial cells with hydrogen peroxide (H₂O₂, 1 mM) to induce oxidative stress. The cell model was divided into an albumin-only treatment group and a glucose-albumin treatment group, with albumin and glucose-albumin each administered at a concentration of 1000 nM.

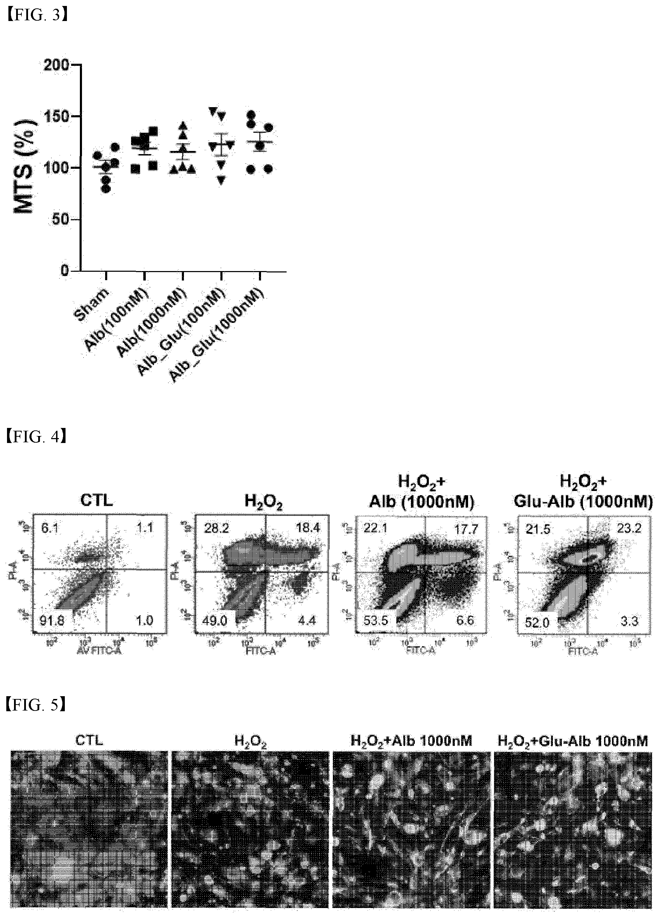

First, MTS analysis was performed to assess cytotoxicity, and the results are shown in FIG. 3. As seen in FIG. 3, no cytotoxicity was observed even with high concentrations of glucose-albumin.

Next, flow cytometry analysis was performed on the AKI cell model treated with albumin or glucose-albumin, and the results are shown in FIG. 4. As shown in FIG. 4, the glucose-albumin treatment group exhibited a significant reduction in apoptosis/necrosis compared to the albumin-only treatment group.

Additionally, fluorescence microscopy analysis was conducted to examine whether glucose-albumin could restore and maintain the function of damaged mitochondria in cells cultured under H₂O₂-induced stress conditions.

As shown in FIG. 5 illustrating the results of the fluorescence microscopy analysis, while fragmented mitochondria were observed under H₂O₂ stress, the glucose-albumin treatment group exhibited restored mitochondrial morphology, appearing in droplet-like or elongated forms.

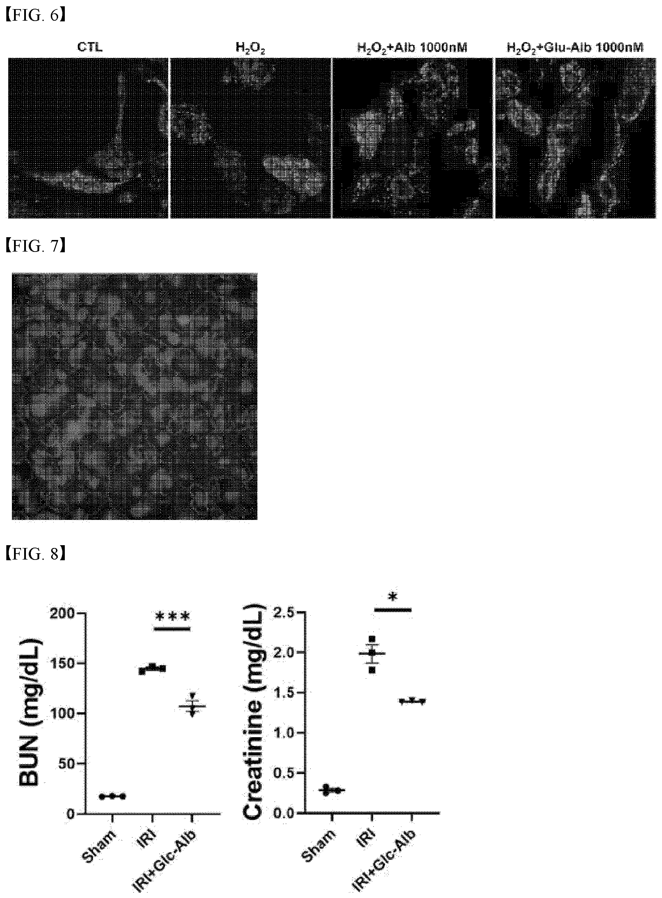

FIG. 6 shows that cells with damaged mitochondria were observed under H₂O₂ stress, while the glucose-albumin treatment group demonstrated recovery of the shape of damaged mitochondria of cells.

These results confirmed that glucose-albumin exhibits a significant mitochondrial morphology restoration effect.

Furthermore, FIG. 7 shows fluorescence microscopy results verifying intracellular expression of glucose-albumin in the AKI cell model. FIG. 7 indicates that glucose-albumin was stably expressed within cells.

These findings demonstrate that glucose-albumin contributes to the recovery of mitochondrial morphology and damaged cells in the kidney.

### Experimental Example 4: Verification of the In Vivo Kidney Function Improvement Effect of the Albumin-Based Nanoplatform

The kidney function and mitochondrial effects of the albumin-based nanoplatform conjugated with the glucosyl group (glucose-albumin) were evaluated in a bilateral ischemia-reperfusion injury (bIRI) mouse model. Glucose-albumin (40 µg/mice) was administered via the tail vein 1 hour before surgery.

First, the BUN (blood urea nitrogen) and creatinine levels of the model were measured, as shown in FIG. 8. Compared to the untreated control group, the glucose-albumin-treated mice showed reduced serum levels of BUN and creatinine, confirming that glucose-albumin is effective in improving kidney function.

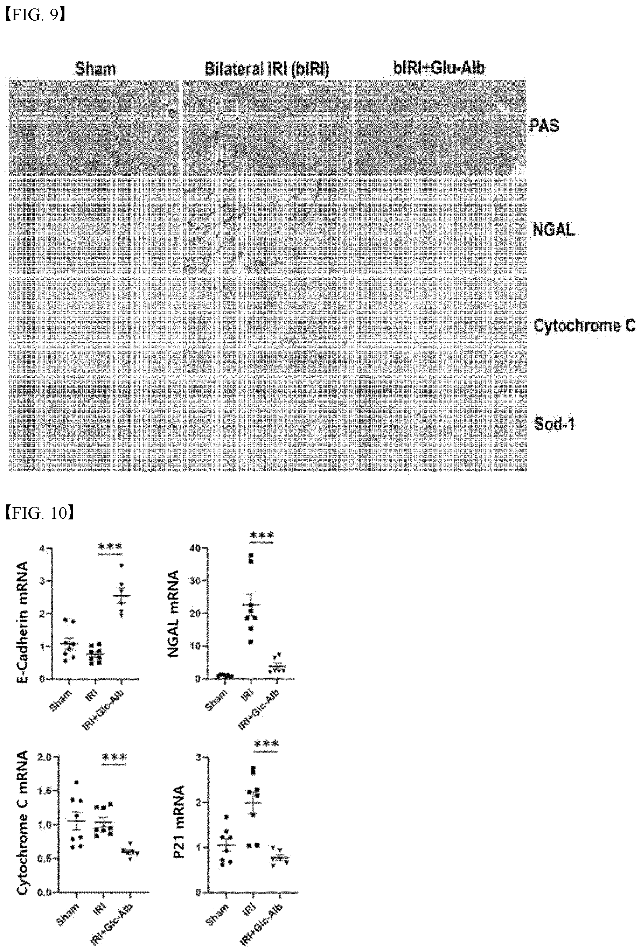

Next, the effects of glucose-albumin on kidney tissue damage and mitochondrial function were analyzed, as shown in FIGS. 9 and 10. Kidney damage markers NGAL, and cytochrome C and p21, which are associated with mitochondrial apoptosis, showed decreased expression by glucose-albumin treatment, and the expression of Sod-1 and E-cadherin, which regulate kidney function, was increased. These results confirmed that glucose-albumin effectively restores the function of damaged kidney tissue and mitochondria.

### Experimental Example 5: Verification of the In Vivo Targeting Ability of the Albumin-Based Nanoplatform

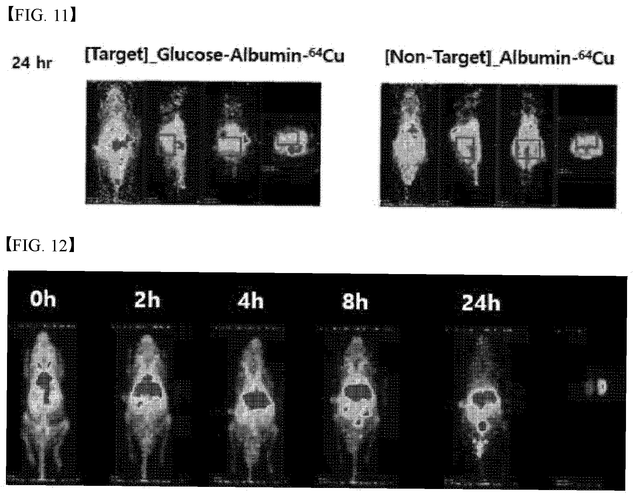

The targeting ability of the albumin-based nanoplatform conjugated with the glucosyl group to diseases and tissues was evaluated in a mouse model by labeling the nanoplatform with isotopes, as shown in FIGS. 11 and 12.

After introducing glucose to albumin using the same method described in Preparation Example 1, isotopes were sequentially introduced in the same manner to obtain a glucosylated albumin nanoplatform (glucose-albumin) labeled with isotopes (⁶⁴Cu). The nanoplatform and a control albumin were administered intraperitoneally (IP) to bIRI mouse models. The fluorescence expression from the isotope was then analyzed to confirm whether the albumin nanoplatform could selectively target diseases and tissues.

As shown in FIG. 11, fluorescence expression was observed within the red box indicating the kidneys in the glucose-albumin-treated group, confirming that glucose-albumin was selectively taken up by the damaged kidneys.

Notably, the glucose-albumin treated group exhibited higher kidney uptake compared to the albumin-treated group, while the albumin-only group displayed increased blood flow at existing inflammatory sites.

These findings confirm that glucosylated albumin demonstrates high targeting efficiency toward damaged kidney tissues. This highlights the potential of the glucosylated albumin nanoplatform as a therapeutic agent for kidney diseases.

Next, the glucose-albumin nanoplatform labeled with isotopes (⁶⁴Cu) was intravenously administered to mice that had undergone unilateral ischemia-reperfusion injury (uIRI) surgery, and fluorescence expression levels were analyzed, as shown in FIG. 12.

As depicted in FIG. 12, the glucose-albumin nanoplatform labeled with isotopes (⁶⁴Cu) was selectively taken up by the damaged kidney, and it was confirmed that it remained in the body even after 24 hours.

Since albumin is not excreted through normal kidneys, these results further confirm that intravenous (IV) administration of glucose-albumin is an effective method for targeting kidney diseases.

While certain embodiments of the present disclosure have been described above, the present disclosure is not limited to these embodiments. Modifications and variations may be made without departing from the spirit and scope of the invention, and such modified and varied forms should also be understood to fall within the technical scope of the present disclosure.

## Claims

1. A nanoplatform for targeting an inflammatory macrophage, obtained by a click chemistry reaction between albumin conjugated with an azide (N₃) or cyclooctyne functional group and a transmitter conjugated with an azide (N₃) or cyclooctyne functional group,
wherein the transmitter comprises a glucosyl group, and
when the albumin is conjugated with the azide functional group, the transmitter is conjugated with the cyclooctyne functional group, and when the albumin is conjugated with the cyclooctyne functional group, the transmitter is conjugated with the azide functional group.

2. The nanoplatform for targeting the inflammatory macrophage of claim 1, wherein the azide or cyclooctyne functional group introduced into the albumin ranges from 1 to 14.

3. The nanoplatform for targeting the inflammatory macrophage of claim 1, wherein the nanoplatform comprises 4 to 8 glucosyl groups.

4. The nanoplatform for targeting the inflammatory macrophage of claim 1, wherein the azide or cyclooctyne functional group is conjugated to the 6th carbon of the glucosyl group.

5. The nanoplatform for targeting the inflammatory macrophage of claim 1, wherein the transmitter further comprises a radioactive isotope, and the radioactive isotope is one or more selected from the group consisting of ³H, ¹¹C, ¹⁸F, ¹⁴Cl, ³²P, ³⁵S, ³⁶Cl, ⁴⁵Ca, ⁵¹Cr, ⁵⁷Co, ⁵⁸Co, ⁵⁹F, ⁶⁴Cu, ⁶⁷Ga, ⁶⁸Ga, ⁸⁹Zr, ⁹⁰Y, ⁹⁹Mo, ^{99m}Tc, ¹¹¹In, ¹³¹I, ¹²⁵I, ¹²⁴I, ¹²³I, ¹⁸⁶Re, ¹⁸⁸Re, ²²⁵Ac, ²¹²Pb, ^{117m}Sn and ¹⁷⁷Lu.

6. The nanoplatform for targeting the inflammatory macrophage of claim 5, wherein the radioactive isotope is labeled by a chelating agent, and the chelating agent is one or more selected from the group consisting of NOTA (1,4,7-triazacyclononane-1,4,7-triacetic acid), DOTA (1,4,7,10-tetraazacyclododecane-1,4,7,10-tetraacetic acid), DFO (3-[6,17-dihyroxy-7,10,18,21-tetraoxo-27-[N-acetylhydroxylamino)-6,11,17,22-tetraazaheptaeicosane]thiourea), DTPA (diethylenetriaminepentaacetic acid), N2S2 (diaminedithiol), p-SCN-Bn-NOTA (2-(4'-isothiocyanatobenzyl)-1,4,7-triazacyclononane-1,4,7-triacetic acid), NODAGA (1,4,7-triazacyclononane,1-glutaric acid-4,7-acetic acid), p-SCN-Bn-DOTA (2-(4'-isothiocyanatobenzyl)-1,4,7,10-tetraazacyclododecane-1,4,7,10-tetraacetic acid), TETA (1,4,8,11-tetraazacyclotetradecane-1,4,8,11-tetraacetic acid), p-SCN-Bn-DTPA (2-(4-isothiocyanatobenzyl)-diethylenetriaminepentaacetic acid), p-SCN-Bn-DFO (1-(4-isothiocyanatophenyl)-3-[6,17-dihyroxy-7,10,18,21-tetraoxo-27-[N-acetylhydroxylamino)-6,11,17,22-tetraazaheptaeicosane]thiourea) and HYNIC (hydrazinonicotinic acid).

7. The nanoplatform for targeting the inflammatory macrophage of claim 1, wherein the transmitter further comprises a fluorescent material, and the fluorescent material is one or more selected from the group consisting of FNR (Ferrodoxin NADP(+) reductase), cyanine-based fluorescent material, TAMRA (tetramethylrhodamine-5-maleimide), Flamma^{®} fluorescent material and ICG (indocyanine green).

8. The nanoplatform for targeting the inflammatory macrophage of claim 1, wherein the nanoplatform selectively targets a macrophage with overexpressed GLUT (Glucose Transporter).

9. A pharmaceutical composition for preventing or treating an inflammatory disease, comprising the nanoplatform for targeting the inflammatory macrophage of any one of claims 1 to 8 as an active ingredient.

10. The pharmaceutical composition of claim 9, wherein the nanoplatform for targeting the inflammatory macrophage selectively targets a damaged tissue.

11. The pharmaceutical composition of claim 9, wherein the nanoplatform for targeting the inflammatory macrophage restores mitochondrial function within the damaged tissue and suppresses cell death.

12. The pharmaceutical composition of claim 9, wherein the inflammatory disease is one or more selected from the group consisting of sepsis, septic shock, inflammatory bowel disease (IBD), peritonitis, inflammatory kidney disease, acute bronchitis, chronic bronchitis, osteoarthritis, enteropathic spondylitis, chronic obstructive pulmonary disease (COPD), rheumatoid arthritis, acute lung injury and bronchopulmonary dysplasia.

13. A pharmaceutical composition for preventing or treating a kidney disease, comprising the nanoplatform for targeting the inflammatory macrophage of any one of claims 1 to 8 as an active ingredient.

14. The pharmaceutical composition of claim 13, wherein the kidney disease is one or more selected from the group consisting of nephritis, pyelonephritis, nephrotic syndrome, kidney cancer, acute pyelonephritis, chronic pyelonephritis, renal tuberculosis, urinary tract infection, urolithiasis, ureteral calculus, acute renal failure, chronic renal failure, diabetic nephropathy, renal fibrosis, chronic glomerulonephritis, rapidly progressive glomerulonephritis, nephrotic syndrome, focal segmental glomerulosclerosis, membranous nephropathy and membranoproliferative glomerulonephritis.
